# EUROPÄISCHE PATENTANMELDUNG

(11) **EP 0 979 815 A1**
(43) Veröffentlichungstag der Anmeldung: **16.02.2000**
(21) Anmeldenummer: 98202692.4
(22) Anmeldetag: 11.08.1998
(51) Int. Cl.: C07C 261/02, C08F 8/00, C08G 73/06

(54) **Ungesättigte Oligophenolcyanate**

(71) Anmelder: LONZA A.G., CH-4002 Basel (CH)
(72) Erfinder: Daum, Ulrich, 4114 Hofstetten (CH); Falchetto, Alessandro, 28030 Monte Crestese (IT)

(57) **Zusammenfassung**

Beschrieben werden ungesättigte Oligophenolcyanate der allgemeinen Formel

[A―]ₙ[B―A―]ₓB[―A]ₘ I.

Darin bedeutet A jeweils eine Gruppe der Formel 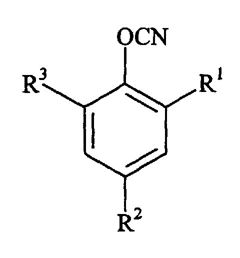 und B jeweils eine Gruppe der Formel 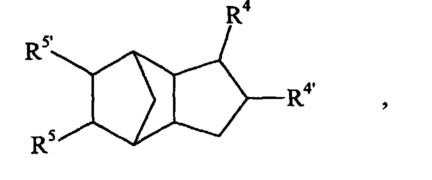 worin R¹, R² und R³ jeweils Wasserstoff oder eine Bindung zu einer Gruppe B bedeuten und entweder eine oder zwei Bindungen zu B vorhanden sind,
und sowohl R⁴ und R^{4'} als auch R⁵ und R^{5'} jeweils entweder gemeinsam eine direkte Bindung oder Wasserstoff und eine Bindung zu einer Gruppe A bedeuten, wobei entweder eine oder zwei Bindungen zu A vorhanden sind. Die Indices n und m sind 0 oder 1, jedoch nicht beide gleichzeitig 1, und x ist eine ganze Zahl von 0 bis 10, wobei wenigstens eine der Zahlen m, n und x von 0 verschieden ist.

Die ungesättigten Oligophenolcyanate können durch Umsetzung der entsprechenden Oligophenole mit Chlorcyan hergestellt werden. Sie besitzen eine niedrige Viskosität und sind auf Grund der vorhandenen Doppelbindungen radikalisch härtbar. Sie eignen sich insbesondere als Matrixmaterial für faserverstärkte Composites und für strahlenhärtbare Lacke und Beschichtungen.

## Beschreibung

Die Erfindung betrifft Oligophenolcyanate der allgemeinen Formel

[A―]ₙ[B―A―]ₓB[―A]ₘ I.

Hierin bedeutet A jeweils eine Gruppe der Formel 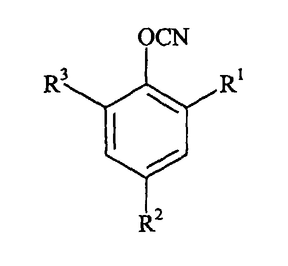 und B jeweils eine Gruppe der Formel

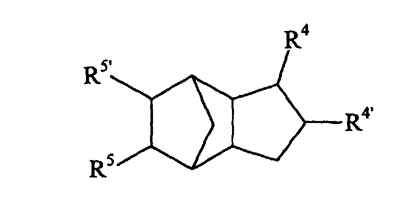

R¹, R² und R³ bedeuten an jeder Gruppe A unabhängig von den anderen jeweils Wasserstoff oder eine Bindung zu einer Gruppe B, mit der Massgabe, dass jede Gruppe A entweder eine oder zwei Bindungen zu B besitzt.
Sowohl R⁴ und R^{4'} als auch R⁵ und R^{5'} bedeuten anjeder Gruppe B unabhängig von den anderen jeweils entweder gemeinsam eine direkte Bindung oder in beliebiger Reihenfolge Wasserstoff und eine Bindung zu einer Gruppe A, mit der Massgabe, dass jede Gruppe B entweder eine oder zwei Bindungen zu A besitzt.
Die Indices m und n sind 0 oder 1 und x ist eine ganze Zahl von 0 bis 10, mit der Massgabe, dass wenigstens eine der Zahlen m, n und x von 0 verschieden ist und m und n nicht beide gleichzeitig 1 sind.

Die Erfindung betrifft insbesondere auch Gemische solcher Verbindungen untereinander und/oder mit solchen Verbindungen der Formel I, in denen abweichend von den obenstehenden Definitionen m und n beide 1 sind.

Gesättigte Oligophenolcyanate der allgemeinen Formel I, in denen abweichend von den Verbindungen gemäss der vorliegenden Erfindung m und n beide 1 sind, sind z. B. aus EP-A-0 147 548 bekannt und werden von der Firma Dow Chemical Co. unter der Bezeichnung XU71787 vertrieben. Diese Verbindungen besitzen keine olefinischen Doppelbindungen und können daher nur durch Cyclotrimerisierung der Cyanatgruppen oder Reaktion mit funktionellen Gruppen anderer Verbindungen polymerisieren. Die Cyclotrimerisierung erfordert die Anwesenheit von Katalysatoren und/oder hohe Temperaturen. Es ist dagegen oft wünschenswert, eine teilweise Aushärtung bzw. Vernetzung beispielsweise durch Bestrahlung bei Raumtemperatur zu erzielen. Ausserdem besitzen diese bekannten Verbindungen eine relativ hohe Viskosität, was für manche Anwendungen ungünstig ist.

Aufgabe der vorliegenden Erfindung war daher, Oligophenolcyanate bereitzustellen, welche eine niedrige Viskosität besitzen und ohne weitere Zusätze bei Raumtemperatur (teil-)polymerisierbar bzw. vernetzbar sind, beispielsweise durch strahleninduzierte Radikalreaktionen.

Erfindungsgemäss wird diese Aufgabe durch die ungesättigten Oligophenolcyanate der Formel I gemäss Patentanspruch 1 gelöst. Diese besitzen im Molekül wenigstens eine olefinische Doppelbindung (R⁴-R^{4'} und/oder R⁵-R^{5'} gemäss Formel I), die eine radikalische Polymerisation erlaubt.
Die olefinischen Doppelbindungen sind in Gruppen der Formel 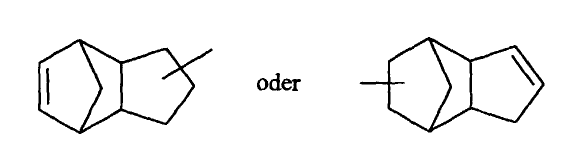 enthalten.

Der Polymerisationsgrad x liegt vorzugsweise zwischen 0 und 5, besonders bevorzugt zwischen 0 und 3.

Die erfindungsgemässen ungesättigten Oligophenolcyanate können hergestellt werden, indem ein Oligophenol der allgemeinen Formel

[A'―]ₙ[B―A'―]ₓB[―A']ₘ II,

worin A' eine Gruppe der Formel 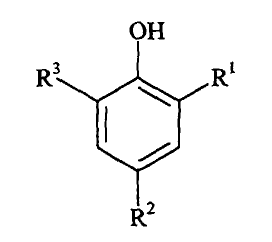 ist und B, R¹, R², R³, R⁴, R^{4'}, R⁵, R^{5'}, m, n und x die in Anspruch 1 genannte Bedeutung haben, in Gegenwart eines tertiären Amins mit Chlorcyan umgesetzt wird. Oligophenole der Formel II sind von der Firma Borden Chemical Inc. unter den Bezeichnungen ESD-X1 bis -X5, ESD-472C und ESD-473C erhältlich. Es handelt sich hierbei um Kondensationsprodukte von Dicyclopentadien (dimeres Cyclopentadien) und Phenol, die als Gemisch isomerer und/oder homologer Verbindungen vorliegen und ausserdem noch Anteile von gesättigten Verbindungen mit m = n = 1 enthalten.

Die Herstellung der erfindungsgemässen Oligophenolcyanate wird vorzugsweise bei einer Temperatur von weniger als 10 °C in einem polaren Lösungsmittel wie beispielsweise Butylacetat und/oder Aceton oder Methylethylketon oder in Mischungen dieser Lösungsmittel durchgeführt. Besonders bevorzugt sind Reaktionstemperaturen unter 0 °C, beispielsweise -10 °C. Vorteilhaft werden pro OH-Äquivalent des Oligophenols II 1,0-1,1 mol tertiäres Amin und 1,0-1,2 mol Chlorcyan eingesetzt. Als tertiäres Amin ist Triethylamin besonders bevorzugt.

Die erfindungsgemässen ungesättigten Oligophenolcyanate weisen bei der Verarbeitungstemperatur eine niedrige Viskosität auf und ergeben Polytriazinharze mit besonders niedriger Dielektrizitätskonstante. Sie eignen sich beispielsweise als Matrixmaterial für die Herstellung von faserverstärkten Composites, insbesondere für Bauteile in der Luft- und Raumfahrttechnik oder Basismaterialien zur Herstellung von Leiterplatten. Wegen ihrer niedrigen Viskosität und der Möglichkeit sie durch energiereiche Strahlen (UV, Röntgen-, γ- oder Elektronenstrahlen) zu polymerisieren, eignen sie sich weiterhin für (Photo-) Lithographielacke, Lötstopmasken für Leiterplatten oder sonstige strahlenhärtbare Lacke und Beschichtungen.

Das folgende Beispiel verdeutlicht die Herstellung der erfindungsgemässen Oligophenolcyanate, ohne dass darin eine Einschränkung zu sehen ist.

### Beispiel

In *n*-Butylacetat/Aceton (*v*/*v* = 80:20) wurde Oligophenol ESD-X3 (Borden Chemical Inc.) zu einer 15%igen Lösung aufgelöst. Die Lösung wurde auf -10 °C abgekühlt und bei dieser Temperatur mit 105% der berechneten Menge Triethylamin und dann innerhalb von 30 min mit 110% der berechneten Menge Chlorcyan versetzt. Nach weiteren 30 min Reaktionsdauer wurde das Gemisch zur Entfernung der gebildeten Ammoniumsalze zweimal bei 30 °C mit Wasser extrahiert und dann zur Entfernung des Lösungsmittels und des Nebenprodukts *N*,*N*-Diethylcyanamid zweimal über einen Fallfilmverdampfer gegeben.
Ausbeute: ca. 100%
Eigenschaften:
Viskosität: 165 mPa·s (bei 125°C)
Umsetzungsgrad (Phenol → Cyanat) >98%
Gelierzeit 25 min (bei 200°C)
Carbamate <1%
*N*,*N*-Diethylcyanamid <2000 ppm

## Patentansprüche

1. Ungesättigte Oligophenolcyanate der Formel
[A―]ₙ[B―A―]ₓB[―A]ₘ I
, wobei A jeweils eine Gruppe der Formel 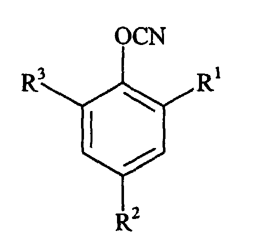 und B jeweils eine Gruppe der Formel 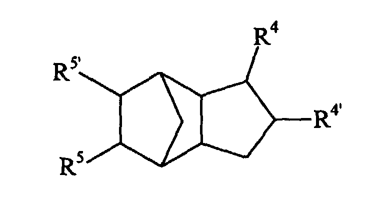 ist,
worin R¹, R² und R³ jeweils Wasserstoff oder eine Bindung zu einer Gruppe B bedeuten, mit der Massgabe, dass jede Gruppe A entweder eine oder zwei Bindungen zu B besitzt;
sowohl R⁴ und R^{4'} als auch R⁵ und R^{5'} jeweils entweder gemeinsam eine direkte Bindung oder Wasserstoff und eine Bindung zu einer Gruppe A bedeuten, mit der Massgabe, dass jede Gruppe B entweder eine oder zwei Bindungen zu A besitzt;
m und n 0 oder 1 sind und x eine ganze Zahl von 0 bis 10 ist, mit der Massgabe, dass wenigstens eine der Zahlen m, n und x von 0 verschieden ist und m und n nicht beide gleichzeitig 1 sind,
sowie deren Gemische untereinander und/oder mit solchen Verbindungen der Formel I, in denen abweichend von den obenstehenden Definitionen n und m beide 1 sind.

2. Oligophenolcyanate nach Anspruch 1, dadurch gekennzeichnet, dass x 0 bis 5 ist.

3. Verfahren zur Herstellung von ungesättigten Oligophenolcyanaten gemäss Anspruch 1, dadurch gekennzeichnet, dass ein Oligophenol der allgemeinen Formel
[A'―]ₙ[B―A'―]ₓB[―A']ₘ II,
worin A' eine Gruppe der Formel 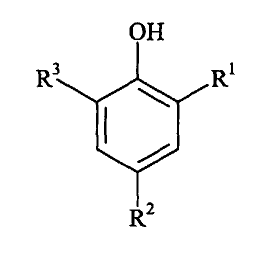 ist und B, R¹, R², R³, R⁴, R^{4'}, R⁵, R^{5'}, m, n und x die in Anspruch 1 genannte Bedeutung haben, in Gegenwart eines tertiären Amins mit Chlorcyan umgesetzt wird.

4. Verwendung der ungesättigten Oligophenolcyanate gemäss Anspruch 1 als Matrixmaterial für faserverstärkte Composites.

5. Verwendung der ungesättigten Oligophenolcyanate gemäss Anspruch 1 als strahlenhärtbare Lacke und Beschichtungen.
